# EUROPÄISCHE PATENTSCHRIFT

(11) **EP 1 247 621 B1**
(45) Veröffentlichungstag und Bekanntmachung des Hinweises auf die Patenterteilung: **23.07.2008**
(21) Anmeldenummer: 02007138.7
(22) Anmeldetag: 28.03.2002
(51) Int. Cl.: B25G 1/10

(54) **Handgriff**
Handle
Poignée

(30) Priorität: 02.04.2001 DE 10116348
(43) Veröffentlichungstag der Anmeldung: 09.10.2002
(73) Patentinhaber: Weber Instrumente GmbH, 78576 Emmingen-Liptingen (DE)
(72) Erfinder: Weber, Helmut, 78576 Emmingen (DE)
(74) Vertreter: Patentanwälte Westphal, Mussgnug & Partner

(56) Entgegenhaltungen:
- EP-A- 0 727 289
- DE-A- 19 803 946
- US-A- 3 762 453
- US-A- 5 897 503

## Beschreibung

Die Erfindung betrifft einen Handgriff für ein medizinisches Werkzeug mit einem inneren Grundkörper, der mit dem Werkzeug verbunden oder verbindbar ist, und mit einem Griffteil.

Es sind zwar verschiedenste Handgriffe bekannt, die aber jeweils für sehr unterschiedliche Anwendungen entwickelt und optimiert sind. Diese Spezialisierung bedeutet, dass auf einem technischen Gebiet bekannte und bewährte Handgriffe nicht ohne weiteres in anderen - z.B. hinsichtlich der mechanischen Belastung oder der hygienischen Anforderungen unterschiedlichen - Gebieten eingesetzt werden können.

Die DE 198 46 681 A1 beschreibt einen Handgriff für einen konventionellen Schraubendreher mit austauschbaren Schraubendreherspritzen. Um diese stets griffbereit zu halten, ist der an sich starre Handgriff hohl ausgeführt und nimmt in seinem Hohlraum zusätzliche Schraubendreherspritzen oder Steckeinsätze auf. Der Hohlraum kann durch eine Trennwand unterteilt sein und ist von außen durch federgetrieben wegschwenkbare Handgriffseitenteile zugänglich. Andere Aspekte, wie z.B. eine bessere Handhabung, sind in der DE 198 46 681 A1 nicht erwähnt.

Aus der DE 198 48 126 A1 ist ein Zusatzhandgriff für handgeführte Elektrowerkzeuge bekannt. Dieser Handgriff umfasst einen Grundkörper, der aus Festigkeitsgründen vorzugsweise aus Metall oder Stahl besteht und an einem Ende eine Stange aufweist. Die Stange verläuft in einem rohrförmigen Griffteil und ist mit diesem verbunden. Das Griffteil muss wegen des üblicherweise rauen und aggressiven Betriebsumfeldes von Elektrowerkzeugen starr ausgebildet sein. Zwischen Griffteil und Grundkörper sind in einem zusätzlichen Bauteil ausgeformte elastische Elemente angeordnet. Diese sorgen dafür, dass beim Betrieb des Elektrowerkzeuges auftretende Schwingungen nicht unmittelbar in die Hand des Bedieners eingeleitet werden. Bei diesem bekannten Handgriff steht also die Vibrationsdämpfung im Vordergrund.

Die DE 199 12 038 C1 beschreibt einen Handgriff für ein medizinisches Instrument. Dieser ist dahingehend optimiert, dass mittels eines beweglichen Griffteils eine Axialbewegung erzeugt werden kann, die in jeder Winkelstellung des Handgriffs auf ein an dem freien Ende des Instruments befindliches Werkzeug übertragen werden kann.

Für chirurgische Instrumente oder Werkzeuge sind auch aus metallischen Werkstoffen bestehende oder aus phenolharzgetränkten Geweben aufgebaute Handgriffe üblich.

Die Herstellung solcher Griffe ist insbesondere bei der letzteren Ausgestaltung aufwendig, weil die endgültige Griffform durch spanabhebende Bearbeitung erzeugt werden muss.

Aus Gründen der Gewichtseinsparung sind auch schon sogenannte Hohlhefte bekannt geworden. Hierzu werden Griffhälften als Metallprägeteilen ein Tiefziehverfahren geformt und miteinander verschweißt. Anschließend werden die so gebildeten Griffteilen poliert, um das gewünschte Oberflächenfinish zu erzielen. Nachteilig hierbei ist der hohe Fertigungsaufwand.

Ein aus Metall hergestellter Handgriff vermittelt zumindest anfangs ein kaltes Griffgefühl, was häufig als unangenehm empfunden wird. Außerdem sind derartige Griffe relativ schwer und unbequem zu greifen und zu handhaben. Dies kann vor allem bei lang andauernden medizinischen Behandlungen eine Ermüdung der taktilen und manuellen Fertigkeiten des Anwenders, z.B. des Chirurgen, hervorrufen.

Als Stand der Technik werden die EP 0159453 A1 und die EP 0791 437 A1 gennant.

Vor diesem Hintergrund ist Aufgabe der vorliegenden Erfindung die Schaffung eines speziell für medizinische, insbesondere chirurgische, Werkzeuge oder Instrumente geeigneten Handgriffs, der sehr angenehm und komfortabel zu handhaben ist, aber dennoch die hohen spezifischen, insbesondere hygienischen, Anforderungen an medizinische Handgriffe erfüllt.

Diese Aufgabe wird erfindungsgemäß gelöst durch einen Handgriff gemaß Anspruch 1.

Ein erster wesentlicher Vorteil des erfindungsgemäßen Handgriffs besteht darin, dass die von dem elastischen Material gebildete äußere Grifffläche ein weiches, angenehmes und ermüdungsfreies Greifen gewährleistet. So ist weder beim erstmaligen Greifen noch bei langandauerndem Halten eine Beeinträchtigung z.B. der Konzentration des Anwenders oder Operateurs zu befürchten. Der erfindungsgemäße Handgriff zeichnet sich außerdem durch einen einfachen, leichten und dennoch robusten Aufbau mit wenigen Einzelteilen aus.

Die Erfindung sieht vor, dass das Griffteil silikonhaltiges Material enthält. Fertigungstechnisch besonders bevorzugt besteht das Griffteil aus Silikongummi. Eine besonders günstige Fertigungssituation ergibt sich, wenn nach einer vorteilhaften Ausgestaltung der Erfindung der Grundkörper unter Bildung des Griffteils unmittelbar mit dem elastischen Material umspritzt ist. Ein geeigneter Silikonwerkstoff ist beispielsweise von der Fa. Wacker unter der Marke Elastosil®R angebotenes Material. Dieses Material ist u.a. für den hier vorgesehenen Einsatzbereich prädestiniert.

Diese Materialien erlauben trotz ihrer weichen und taktil angenehmen Beschaffenheit eine Dampfsterilisierung (bei ca. 136° C) und sind säurebeständig sowie physiologisch unbedenklich.

Darüber hinaus hat es sich gezeigt, dass beim Aufspritzen des Silikon-Kautschuk-Mantels auf den Grundkörper eine hochfeste und intensive Verbindung entsteht, die eine spaltfreie und dichte Verbindung der beiden Werkstoffe ermöglicht. Die bei den bisher üblichen Griffen auftretenden Spalte in Folge der unterschiedlichen Ausdehnungskoeffizienten der beiden Werkstoffe treten nicht mehr auf. Darüber hinaus wird zuverlässig das Eindringen von Schmutz an der Grenzfläche zwischen beiden Werkstoffen in Folge Kapilarwirkung zuverlässig verhindert. Optional kann durch zusätzliches Verschweißen mittels Laser eine Abdichtung an den Verbindungsstellen zwischen dem eigentlichen Instrument und dem Griff erzielt werden.

Ein besonders angenehmes Griffgefühl läßt sich mit einem Silikon-Kaubchuk erzielen, der eine Shore-Härte im Bereich von 75 bis 85 besitzt.

Ausführungsbeispiele der Erfindung werden nachfolgend anhand einer Zeichnung näher erläutert; es zeigen:
- Figur 1: den Grundkörper eines ersten Handgriffs,
- Figuren 2 und 3: den ersten Handgriff im Längsschnitt bzw. in Gesamtansicht;
- Figur 4: den Grundkörper eines erfindungsgemäßen Handgriffs vor der Montage,
- Figuren 5 und 6: den erfindungsgemäßen Handgriff im Längsschnitt bzw. in Gesamtansicht.

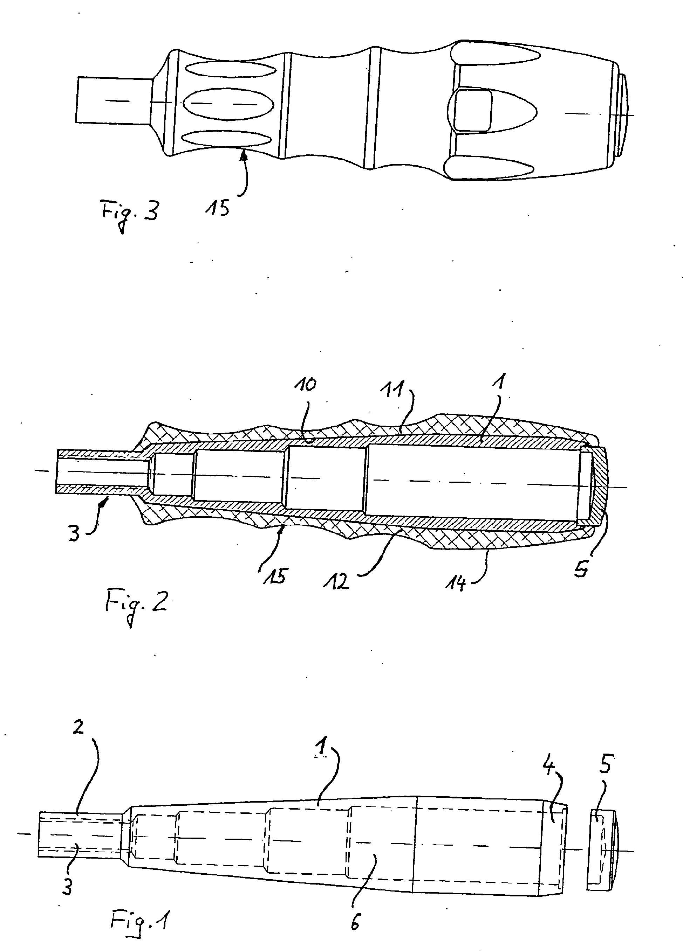

Figur 1 zeigt einen Grundkörper 1 eines ersten Handgriffs. Der Grundkörper 1 ist rohrförmig und im wesentlichen hohlzylindrisch ausgebildet. Er hat einen ersten Endbereich 2 mit einer Verbindungseinrichtung 3, an der in nicht näher dargestellter Weise ein medizinische Werkzeug oder Instrument befestigt werden kann. Die Verbindungseinrichtung 3 kann als Steckverbindung z.B. in Form eines Vielkantes ausgebildet sein und mit einer korrespondierenden Form des Werkzeugs formschlüssig verbindbar sein. Der Grundkörper 1 erweitert sich von seinem ersten Endbereich 2 zu seinem zweiten Endbereich 4, der an sich geschlossen sein kann oder - wie dargestellt - durch Aufsatz einer Kappe 5 verschließbar ist. Die Kappe 5 kann für einen späteren Zugang in einen inneren Hohlraum 6 des Grundkörpers 1 entfernbar sein oder ist als Schlagfläche für einen Hammer ausgebildet. Der Durchmesser des Hohlraums 6 erweitert sich stufenweise von dem ersten Endbereich 2 zum zweiten Endbereich 4. Damit weist der Grundkörper 1 insgesamt nur wenig Material und dadurch ein sehr geringes Eigengewicht auf.

Figur 2 stellt den ersten Handgriff im Längsschnitt dar. Der Grundkörper 1 ist hier bereits an seiner Außenwand 10 bis auf seinen Endbereich 2 und die Kappe 5 vollständig mit einem elastischen Material 11, z.B. einem silikonhaltigen Material, umspritzt. Dazu kann der Grundkörper 1 in fertigungstechnisch einfacher Weise in eine Spritzform eingebracht werden, die anschließend unter Druck z.B. mit noch fließfähigem Silikongummi gefüllt wird. Ein hierzu geeigneter Silikonwerkstoff ist das unter der Marke Elastosil®R von der Fa. Wacker vertriebene Material.

Mit Vernetzung der Silikonstruktur entsteht dann ein Griffteil 12, das deutlich weicher als z.B. übliche Kunststoffgriffteile aus Duroplasten oder Thermoplasten ist. Damit entsteht ein dampfsterilisierbarer Handgriff, der säurebeständig und physiologisch unbedenklich ist. Das Griffteil 12 bildet mit seiner Außenseite 14 eine weiche Grifffläche 15 und ermöglicht ein weitgehend ermüdungsfreies Handhaben des Griffs bzw. des damit verbundenen medizinischen Instruments oder Werkzeugs.

Figur 3 zeigt der Vollständigkeit halber die Außenansicht eines ersten Handgriffs und dessen ergonomisch geformte Grifffläche 15.

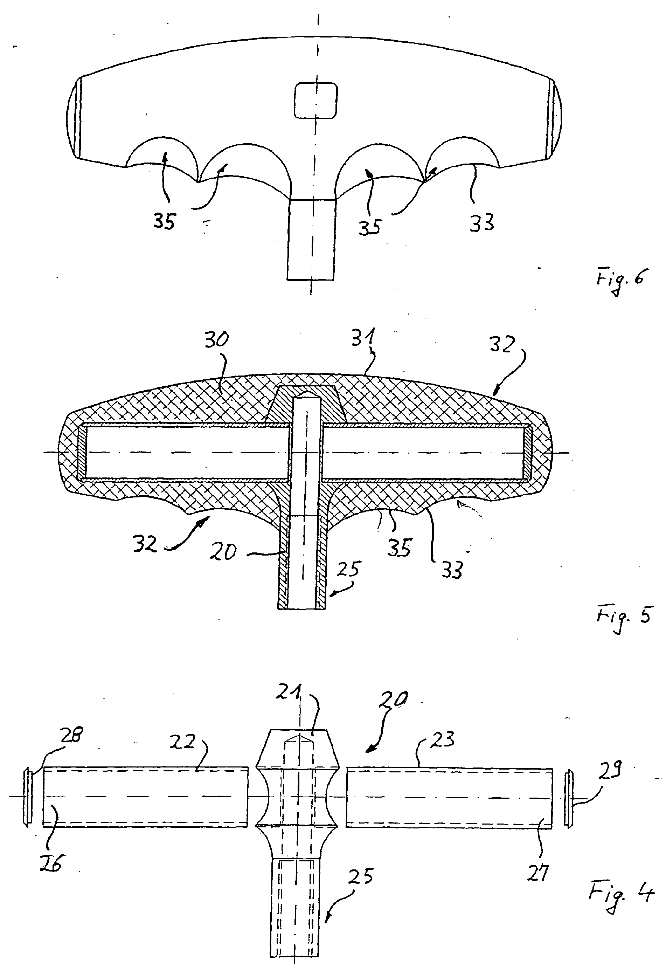

Der in den Figuren 5 und 6 im Längsschnitt bzw. in Gesamtansicht gezeigte erfindungsgemäße Handgriff unterscheidet sich von dem ersten Ausführungsbeispiel im wesentlichen durch seine durch den Grundkörper vorgegebene äußere Form.

Figur 4 zeigt einen Grundkörper 20, der aus einem zentralen Ansatzstück 21 und zwei beiderseits der Längssymmetrieachse des Ansatzstücks 21 angesetzte Rohrstücken 22, 23 zusammengesetzt ist. Die genannten Teile können z.B. als Stahlteile ausgebildet sein, die aus einem handelsüblichen Endloshohlprofil aus Stahl der Bezeichnung 1.4301 in der jeweils erforderlichen Länge abgeschnitten und durch Schweißen zu einer T-Form verbunden sind (Figur 5). Das Ansatzstück 21 dient mit seinem freien Endbereich 25 zur (ggf. lösbaren) Verbindung mit einem nicht näher dargestellten medizinischen Werkzeug oder Instrument. Diese Verbindung kann als formschlüssige Steckverbindung realisiert sein. Die äußeren Enden 26, 27 der Rohrstücken 22, 23 sind durch je einen Stopfen 28, 29 verschließbar.

Figur 5 stellt den erfindungsgemäßen Handgriff im Längsschnitt dar. Der hohle Grundkörper 20 ist hier bereits montiert und bis auf den freien Endbereich 25 vollständig mit Silikon umgeben 30. Auch der Grundkörper 20 kann dazu in eine Spritzform eingebracht werden, die anschließend unter Druck z.B. mit noch fließfähigem Silikongummi gefüllt wird. Das mit Vernetzung der Silikonstruktur entstehende Griffteil 31 hat eine Außenseite 32 mit einer weichen Grifffläche 33, die für eine griffige und ermüdungsfreie Handhabung sorgt und dazu eine grifffingerangepasste Struktur 35 hat.

Figur 6 zeigt der Vollständigkeit halber die Außenansicht des erfindungsgemäßen Handgriffs und dessen Grifffläche 33 mit der Griffstruktur 35.

### Bezugszeichenliste:

- 1: Grundkörper
- 2: erster Endbereich
- 3: Verbindungseinrichtung
- 4: zweiter Endbereich
- 5: Kappe
- 6: Hohlraum
- 10: Außenwand
- 11: silikonhaltiges Material
- 12: Griffteil
- 14: Außenseite
- 15: Grifffläche
- 20: Grundkörper
- 21: Ansatzstück
- 22, 23: Rohrstücke
- 25: freier Endbereich
- 26, 27: äußere Enden der Rohrstücke 22, 23
- 28, 29: Stopfen
- 30: Silikon
- 31: Griffteil
- 32: Außenseite
- 33: Grifffläche
- 35: Griffstruktur

## Patentansprüche

1. Handgriff für ein medizinisches Werkzeug, mit
- einem Grundkörper (1), der mit dem Werkzeug verbunden oder verbindbar ist, und
- einem Griffteil (12), das mit dem Grundkörper (1) verbunden ist, wobei das Griffteil (12) aus einem elastischen Material (11) besteht, das zumindest teilweise an einer äußeren Grifffläche (15) endet,
- wobei das Griffteil (15) silikonhaltiges Material enthält, und wobei der Grundkörper (1) zumindest teilweise aus Abschnitten (22, 23) eines Endlos-Hohlprofils aufgebaut ist, welche zu einer T-Form verbunden sind und bis auf einen freien Endbereich (25) vollständig mit dem silikonhaltigen Material umgeben sind.

2. Handgriff nach Anspruch 1,
- wobei das Griffteil (15) aus Silikongummi besteht.

3. Handgriff nach Anspruch 1, oder 2,
- wobei das Griffteil (12) zumindest im Bereich der äußeren Grifffläche (15) eine Shore-Härte zwischen 75 und 85 aufweist.

4. Handgriff nach einem der Ansprüche 1 bis 3,
- wobei der Grundkörper (1) unter Bildung des Griffteils (12) unmittelbar mit dem elastischen Material (11) umspritzt ist.

5. Handgriff nach einem der Ansprüche 1 bis 4,
- wobei der Grundkörper (1) ein Hohlkörper ist.

## Claims

1. A handle for a medical tool, having
- a main body (1), which is connected or can be connected to the tool, and
- a grip (12) connected to the main body (1), the grip (12) being made from an elastic material (11) that ends at least partially at an outer grip surface (15),
- wherein the grip (15) contains silicon-based material, and wherein the main body (1) is at least partially constructed from portions (22, 23) of an endless hollow profile, which are connected to form a T shape and are completely surrounded with the silicon-based material except for a free end region (25) .

2. A handle according to Claim 1,
- wherein the grip (15) is made from silicon rubber.

3. A handle according to Claim 1 or 2,
- wherein the grip (12) comprises a Shore hardness of between 75 and 85 at least in the region of the outer grip surface (15).

4. A handle according to one of Claims 1 to 3,
- wherein the main body (1) is directly extrusion-coated with the elastic material (11) with the formation of the grip (12).

5. A handle according to one of Claims 1 to 4,
- wherein the main body (1) is a hollow body.

## Revendications

1. Poignée pour un outil médical comprenant
- un corps de base (1) relié à l'outil ou susceptible d'être relié à l'outil et
- une partie de préhension (12) reliée au corps de base (1), la partie de préhension (12) étant en une matière élastique (11), se terminant au moins en partie par une surface extérieure de préhension (15),
- la partie de préhension (12) contenant un matériau avec du silicone et
- le corps de base (1) étant formé au moins partiellement de segments (22, 23) d'un profil creux sans fin, relié à une forme en T et entouré jusqu'à une zone d'extrémité libre (25), complètement avec la matière contenant du silicone.

2. Poignée selon la revendication 1,
dans laquelle la partie de préhension (15) est en caoutchouc de silicone.

3. Poignée selon la revendication 1 ou 2,
dans laquelle
la partie de préhension (12) présente au moins dans la zone de surface extérieure de préhension (15), une dureté shore comprise entre 75 et 85.

4. Poignée selon l'une des revendications 1 à 3,
dans laquelle
le corps de base (1) est surmoulé par injection directement avec la matière élastique (11) pour former la partie de préhension (12).

5. Poignée selon l'une des revendications 1 à 4,
dans laquelle
le corps de base (1) est un corps creux.
